Europäisches Patentamt

European Patent Office

Office européen des brevets

(19)

(11) Publication number: **0 070 459**
A2

# EUROPEAN PATENT APPLICATION

(12)

(21) Application number: 82106090.2

(22) Date of filing: **07.07.82**

(51) Int. Cl.³: **A 61 B 17/36**

(30) Priority: **13.07.81 JP 108109/81**

(43) Date of publication of application: **26.01.83**
**Bulletin 83/4**

(84) Designated Contracting States: **DE FR GB SE**

(71) Applicant: **SUMITOMO ELECTRIC INDUSTRIES
LIMITED, No. 15, Kitahama 5-chome Higashi-ku,
Osaka-shi Osaka (JP)**

(72) Inventor: **Takenaka, Shinya c/o Sumitomo Electric, Ind.
Ltd. Osaka Works, No. 1-3 Shimaya 1-chome,
Konohana-ku, Osaka-shi, Osaka (JP)**
Inventor: **Sunago, Katsuyoshi c/o Sumitomo Electric,
Ind. Ltd. Osaka Works, No. 1-3 Shimaya 1-chome,
Konohana-ku, Osaka-shi, Osaka (JP)**

(74) Representative: **Patentanwälte Henkel, Pfenning, Feiler,
Hänzel & Meinig, Möhlstrasse 37,
D-8000 München 80 (DE)**

(54) Hand piece for laser knife.

(57) A hand piece for a laser knife includes a first holder with a gripping end portion and a second holder with a gripping end portion, and the holders are joined together in a manner such that the gripping end portions are elastically spaced in the manner of tweezers. A laser beam transmitting optical fiber is mounted on the gripping end portion of the first holder in a manner such that the emergent end of the optical fiber confronts the gripping end portion of the second holder, which is adapted to intercept the laser beam.

# HAND PIECE FOR LASER KNIFE

## BACKGROUND OF THE INVENTION

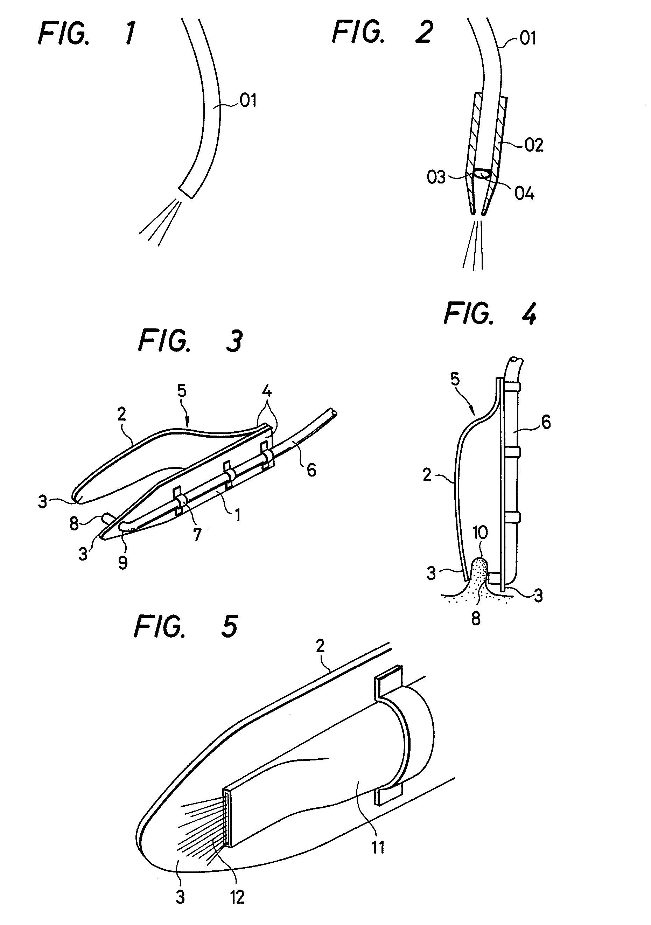

This invention relates to a hand piece for a laser knife. Heretofore, in a laser knife using an energy beam transmitting optical fiber (hereinafter referred to merely as "a fiber"), a covered fiber 01 as shown in Fig. 1 is used directly. Alternatively, in such a laser knife, the front end portion of a fiber 01 is inserted into a straight tube 02, and a lens 04 is provided at the emergent end 03 of the fiber 01, so tht the laser beam emerging from the end 03 is applied to one point of the target part. In the former case, the fiber 01 is not reinforced. Accordingly, the fiber is readily bent even by a small force. Furthermore, since it is hazardous to hold the front end portion of the fiber 01, it is difficult to correctly apply the laser beam to the diseased tissue. On the other hand, in the latter case, the fiber 01 is inserted into the straight tube 02. Accordingly, the flexibility of the fiber is limited, and the direction of irradiation is limited to the axial direction of the straight tube 02. Accordingly, the range of applicability of the device is reduced by as much. Furthermore, when the power of the laser beam is high with respect to the thickness of the diseased tissue to be removed, or when it is necessary to apply the beam to the body part several times in order to remove the diseased tissue, the laser beam may pass through the diseased region to damage normal cells or tissues in the irradiation direction.

In severing tissues, in order to facilitate the surgical operation, a pair of tweezers held by one hand is normally used to grip the tissues to be removed, while a laser knife held by the other hand is used to sever the tissue; i.e., both hands are used. In this case, it is difficult to correctly apply the laser beam to the tissues to be removed. If the hands shake even slightly, normal cells around the diseased area may be damaged.

## SUMMARY OF THE INVENTION

Accordingly, an object of this invention is to provide a hand piece for a laser knife in which the above-described difficulties have been eliminated; i.e., wherein the laser beam is applied to only a desired area, and adjacent normal cells are not affected by the application of the laser beam.

The foregoing object and other objects as well as the specific features of the invention will become more apparent from the following detailed description when read in conjunction with the accompanying drawings, in which like parts are designated by like reference numerals.

## BRIEF DESCRIPTION OF THE DRAWINGS

Figs. 1 and 2 are explanatory diagrams showing examples of a conventional laser knife hand piece;

Fig. 3 is a perspective view showing a first example of a laser knife hand piece according to the invention;

Fig. 4 is an explanatory diagram showing the use of the hand piece shown in Fig. 3; and,

Fig. 5 is an enlarged perspective view showing a part of a second example of the laser knife hand piece according to the invention.

DETAILED DESCRIPTION OF THE PREFERRED EMBODIMENTS

A first example of a laser knife hand piece according to the invention is in the form of a pair of tweezers as shown in Fig. 3. The hand piece 5 comprises a first holder 1 and a second holder 2, the rear end portions 4 of which are joined together in such a manner that the gripping end portions 3 thereof are elastically closely spaced from each other. An optical fiber 6 is fixedly secured to the outer wall of the first holder with fasteners 7. The front end portion of the optical fiber 6 is inserted into a hole 9 cut in the first holder 1 in a manner such that the emergent end of the fiber 6 confronts the gripping end portion 3 of the second holder 2.

The first example described above may be modified by replacing the first holder with a bent tube. After an optical fiber is inserted into the bent tube, the tube is then coupled to the second holder in a manner such that the emergent end of the fiber and the gripping end portion of the second holder are elastically closely spaced. As a further modification, an optical fiber may be secured to a hand piece in the form of a clothespin. When necessary, the gripping portions of the hand piece are then manually operated to elastically grip the diseased tissues.

In cutting the diseased tissues with the hand piece described above, the diseased region 10 is gripped and is pulled upwardly with the gripping end portion 3 of the second holder 2 and the emergent end 8 of the fiber 6. In this condition, the laser beam is then applied to sever the tissues 10 and remove the latter.

The laser beam can be sufficiently intercepted by increased the area of the gripping end portion 3 of the second holder 2. However, if the gripping end portion 3 has a mirrored or smoothened surface, the laser beam is reflected thereby. Such reflection is of course hazardous. In order to prevent the same, it is necessary to roughen the surface of the gripping end portion 3 to thereby irregularly reflect the laser beam, or to subject the gripping end portion 3 to a surface treatment so that the gripping end portion 3 is suitably colored to absorb the laser beam to an extent.

A second example of the laser knife hand piece according to the invention is as shown in Fig. 5. In this example, a water supplying tube 11 mounted on the second holder 2 has an opening near the gripping end portion 3 of the second holder 2, so that a liquid or physiological salt solution adapted to intercept the laser beam is applied to the surface of the gripping end portion 3 to which the laser beam is applied. In this case, not only is the gripping end thus cooled, but also the laser beam is absorbed. Thus, the laser knife hand piece is excellent from a safety standpoint.

The second example of the hand piece may be modified by providing a cooling medium circulating path in the hand piece itself. Also, the second holder may be provided in the form of a pipe through which cooling solution flows, and the cooling solution may be discharged through small holes cut in the gripping end portion.

The laser knife hand piece according to the invention is provided in the form of a pair of tweezers as described above. Therefore, the diseased part alone can readily be gripped with the hand piece, and accordingly it can be safely cut and removed. When the laser beam passes through the diseased tissue, it is intercepted by the gripping end portion, which confronts the emergent end of the optical fiber, so that adjacent normal cells are protected from damage due to the laser beam.

WHAT IS CLAIMED IS:

1.      A hand piece for a laser knife, comprising, a first holder having a gripping end portion, and a second holder having a confronting gripping end portion, said first and second holders being joined in a manner such that said gripping end portions are elastically closely spaced, and

a laser beam transmitting optical fiber mounted on said gripping end portion of said first holder is a manner such that an emergent end of said optical fiber confronts said gripping end portion of said second holder, said second holder including means capable of intercepting said laser beam.

2.      A hand piece as claimed in claim 1, said means for intercepting said laser beam comprising a surface treated area on said confronting gripping end portion for partially absorbing said laser beam.

3.      A hand piece as claimed in claim 1, said means for intercepting said laser beam comprising a roughened area adapted to scatter said beam.

4.      A hand piece as claimed in claim 1, said means for intercepting said laser beam comprising a conduit arranged on said second holder and including an outlet, and a laser beam absorbing fluid flowing in said conduit.

5.      A hand piece as claimed in claim 4, said conduit comprising a circulating path for a cooling medium.

6.      A hand piece as claimed in claim 1, further comprising cooling means including a circulating path for a cooling medium, and forming at least a part of said second holder.

7.     A hand piece as claimed in claim 1, said first holder comprising a bent tube receiving said fiber.

8.     A hand piece as claimed in claim 1, said gripping end portion of said first holder including said emergent end portion of said optical fiber.

FIG. 1

FIG. 2

FIG. 3

FIG. 4

FIG. 5